(19) Europäisches Patentamt
European Patent Office
Office européen des brevets

(11) Veröffentlichungsnummer: **0 435 085 A1**

(12) **EUROPÄISCHE PATENTANMELDUNG**

(21) Anmeldenummer: **90124076.2**

(22) Anmeldetag: **13.12.90**

(51) Int. Cl.5: **C07C 311/17**, C07C 311/13, C07C 303/38, A61K 31/18, C07D 207/16, C07D 211/60

(30) Priorität: **23.12.89 DE 3942923**

(43) Veröffentlichungstag der Anmeldung: **03.07.91 Patentblatt 91/27**

(84) Benannte Vertragsstaaten: **AT BE CH DE DK ES FR GB GR IT LI LU NL SE**

(71) Anmelder: **BOEHRINGER MANNHEIM GMBH**
**Sandhofer Strasse 116**
**W-6800 Mannheim 31(DE)**

(72) Erfinder: **Witte, Ernst-Christian, Dr. rer. nat.**
**Beethovenstrasse 2**
**W-6800 Mannheim 1(DE)**
Erfinder: **Stegmeier, Karlheinz, Dr. rer. nat.**
**Kirchbergstrasse 17**
**W-6148 Heppenheim(DE)**
Erfinder: **Doerge, Liesel, Dr. med.**
**Schwalbenstrasse 26**
**W-6840 Lampertheim(DE)**

(54) **Phenoxyalkylcarbonsäure-Amide, Verfahren zu ihrer Herstellung sowie diese Verbindungen enthaltende Arzneimittel.**

(57) Phenoxyalkylcarbonsäureamide der Formel I

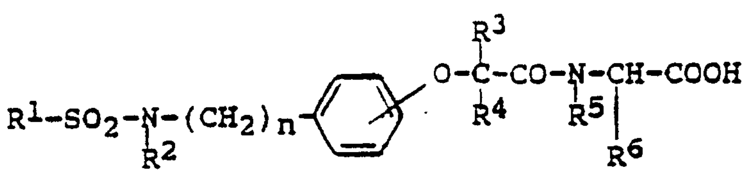

(I)

in welcher die Sulfonylaminoalkylgruppe in ortho-, meta- oder para-Stellung zur Phenoxyalkylcarbonamid-Gruppe steht und in welcher

- $R^1$ eine Aryl-, Aralkyl oder eine Aralkenylgruppe bedeutet, deren Arylrest jeweils ein- oder mehrfach durch Halogen, Cyan, Alkyl, Trifluormethyl oder Alkoxy substituiert sein kann,
- $R^2$ Wasserstoff, eine Alkyl- oder eine Acylgruppe darstellt,
- $R^3$ und $R^4$, die gleich oder verschieden sein können, Wasserstoff oder eine niedere Alkylgruppe und
- n die Zahlen 1-3

bedeuten und worin

- $R^6$ Wasserstoff oder eine niedere Alkylgruppe mit 1-4 C-Atomen bedeutet, welche ggf. endständig durch Carboxyl, Aminocarbonyl, Alkoxycarbonyl, durch Alkylthio-, Hydroxyl, Phenyl oder durch eine Imidazol-4-ylgruppe substituiert ist und
- $R^5$ Wasserstoff bedeutet, oder zusammen mit $R^6$ eine Alkylenkette mit 3 oder 4 C-Atomen bildet,

deren physiologisch verträgliche Salze, Ester und Amide und optische Isomere, Verfahren zu ihrer Herstellung

EP 0 435 085 A1

sowie Arzneimittel zur Behandlung von Herz- und Kreislauferkrankungen.

# NEUE PHENOXYALKYLCARBONSÄURE-AMIDE, VERFAHREN ZU IHRER HERSTELLUNG SOWIE DIESE VERBINDUNGEN ENTHALTENDE ARZNEIMITTEL

Die vorliegende Erfindung betrifft am Phenyl substituierte Phenoxyalkylcarbonsäureamide, Verfahren zu ihrer Herstellung sowie Arzneimittel, die diese Verbindungen enthalten.

In der DE-OS 2809377 sind Phenoxyalkylcarbonsäuren und einige Amide mit lipidsenkender und die Thrombozytenaggregation hemmender Wirkung beschrieben, die in 4-Stellung substituiert sind.

In der DE-OS 36 10 643 sind Phenoxyalkylcarbonsäuren und einige Amide mit lipidsenkender und die Thrombozytenaggregation hemmender Wirkung beschrieben, die in 2- und 3-Stellung substituiert sind.

Es wurde nun überraschenderweise gefunden, daß substituierte Phenoxyalkylcarbonsäure-Amide, bei denen die Aminkomponente eine Aminosäure darstellt, eine ausgezeichnete lipidsenkende und thromboxan-A2-antagonistische Wirkung zeigen.

Die vorliegende Verbindung betrifft daher neue Phenoxyalkylcarbonsäureamide der allgemeinen Formel I

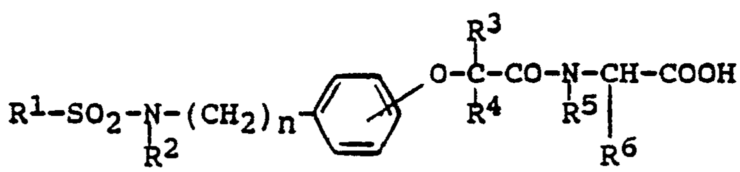

(I)

in welcher die Sulfonylaminoalkylgruppe in <u>ortho-</u>, <u>meta-</u> oder <u>para</u>-Stellung zur Phenoxyalkylcarbonamid-Gruppe steht und in welcher:

| | |
|---|---|
| $R^1$ | eine Aryl-, Aralkyl oder eine Aralkenylgruppe bedeutet, deren Arylrest jeweils ein- oder mehrfach durch Halogen, Cyan, Alkyl, Trifluormethyl oder Alkoxy substituiert sein kann, |
| $R^2$ | Wasserstoff, eine Alkyl- oder eine Acylgruppe darstellt, |
| $R^3$ und $R^4$, | die gleich oder verschieden sein können, Wasserstoff oder eine niedere Alkylgruppe und |
| n | die Zahlen 1-3 |
| | bedeuten und worin |
| $R^6$ | Wasserstoff oder eine niedere Alkylgruppe mit 1-4 C-Atomen bedeutet, welche ggf. endständig durch Carboxyl, Aminocarbonyl, Alkoxycarbonyl, durch Alkylthio-, Hydroxyl, Phenyl oder durch eine Imidazol-4-ylgruppe substituiert ist und |
| $R^5$ | Wasserstoff bedeutet, oder zusammen mit $R^6$ eine Alkylenkette mit 3 oder 4 C-Atomen bildet. |

Es handelt sich also um Phenoxycarbonsäureamide mit Aminosäuren als Aminkomponente. Beansprucht werden außer den Amid-säuren auch deren physiologisch unbedenkliche Salze, Ester und Amide. Da die Aminkomponenten der Phenoxycarbonsäureamide asymmetrische Kohlenstoffatome enthalten, sind auch deren reine optische Isomere (Enantiomere) bzw. deren Gemische (Razemate) Gegenstand der Erfindung.

Die neuen Verbindungen der allgemeinen Formel I zeigen eine ausgezeichnete antagonistische Wirkung gegenüber Thromboxan $A_2$ sowie gegen Prostaglandin-endoperoxide. Sie inhibieren die Aktivierung von Blutplättchen und anderen Blutzellen und verhindern die Konstriktion der glatten Muskulatur von Bronchien und Blutgefäßen sowie die Kontraktion von Mesangiumzellen und ähnlichen Zellen mit kontraktilen Eigenschaften.

Diese Wirkung machen sie zu wertvollen Heilmitteln zur Behandlung von cardiovasculären Erkrankungen wie dem akuten Herz- und Hirninfarkt, cerebraler und coronarer Ischämie, Migräne, peripherer arterieller Verschlußkrankheit sowie venöser und arterieller Thrombosen. Weiterhin kann ihre frühzeitige Anwendung das Auftreten von Organschäden bei Schockpatienten günstig beeinflussen. Sie sind weiter geeignet zur Verhinderung des Thrombozyten- und Leukozytenabfalls bei Eingriffen mit extracorporalem Kreislauf und bei der Hämodialyse. Ihr Zusatz zu Thrombozytenkonzentraten stabilisiert die Blutplättchen und steigert somit die Lagerfähigkeit der Konserven.

Da Thromboxan beim Asthma bronchiale ein Mediator der entzündlichen Reaktion ist, kann durch die Anwendung dieser Thromboxan-Rezeptor-Blocker vor allem die für das chronische Asthma charakteristische

Hyperreaktivität abgeschwächt oder sogar beseitigt werden.

Die neuen Thromboxan-Rezeptor-Blocker sind weiter protektiv wirksam bei Gastritis und Ulcusneigung und somit zur Rezidivprophylaxe derselben einsetzbar. In einem Modell der experimentellen akuten Pankreatitis konnte der Verlauf durch den Einsatz eines Thromboxan-Antagonisten gebessert werden. Es ist somit zu erwarten, daß zumindest bestimmte Formen der akuten Pankreatitis beim Menschen in ihrer Prognose durch den Einsatz dieser Thromboxan-Antagonisten verbessert werden können.

Zusätzlich vermögen sie den Acetateinbau in Cholesterin zu hemmen und eignen sich daher auch zur Behandlung von Fettstoffwechselerkrankungen, die mit gesteigerter Cholesterinsynthese einhergehen. Besonders zu betonen ist ihr ausgeprägter antiatherogener Effekt bei erhöhten Serumcholesterinwerten, der sich in einer Reduktion der Plaquebildung besonders in den Coronararterien und in der Aorta zeigt.

Da der Diabetes mit einer gesteigerten Thromboxanbildung einhergeht, lassen sich durch die chronische Anwendung dieser Thromboxan-Antagonisten die typischen Spätschäden an Gefäßen der Niere und am Auge in ihrer Entwicklung verzögern oder sogar verhindern.

Auch bei einer Reihe von immunologisch oder nicht-immunologisch bedingten Nierenerkrankungen, wie z.B. Glomerulonephritis, akutem Nierenversagen, Transplantatabstoß und durch nephrotoxische Substanzen bedingter Nierenschädigung, wurde eine vermehrte Ausscheidung von Thromboxan B2 im Urin beobachtet. Bei diesen Erkrankungen ist eine Intervention mit den neuen Thromboxan-Antagonisten im Hinblick auf die Erhaltung der Nierenfunktion erfolgsversprechend.

Da bei Tumorzellen eine vermehrte Thromboxansynthese nachgewiesen wurde und gleichzeitig die Proliferation dieser Zellen durch die Gabe von Thromboxan-Antagonisten gehemmt werden konnte, stellen diese eine wirkungsvolle adjuvante Therapie dar.

Bei der pathologischen Schwangerschaft wird eine Störung des Gleichgewichts der Prostaglandine als ursächlich angesehen. Daher kann durch Blockade der Thromboxan- und PGF2alpha-Rezeptoren insbesondere die vorzeitige Wehentätigkeit unterbrochen werden und bei Schwangerschaftsgestose bzw. Eklampsie ein günstigerer Verlauf erzielt werden. Daneben lassen sich hierdurch auch die prostaglandinbedingten Symptome der Dysmenorrhoe und des prämenstruellen Syndroms therapieren.

Als "Arylrest", allein oder in Verbindung mit einer Alkyl- oder Alkenylkette, sind in allen Fällen aromatische Kohlenwasserstoffe mit 6-14 C-Atomen, insbesondere der Phenyl-, der Biphenylyl-, der Naphthyl- und der Fluorenylrest zu verstehen. Diese Arylreste können in allen möglichen Positionen ein-, zwei- oder dreifach substituiert sein, wobei als Substituenten Halogen, $C_1$-$C_6$ Alkyl, $C_1$-$C_6$ Alkoxy, Trifluormethyl oder Cyan in Frage kommen. Bevorzugt ist der Phenylrest, der durch Halogen, bevorzugt Chlor und Brom; Methoxy, Methyl oder Trifluormethyl substituiert sein kann.

Unter den Alkyl- und Alkoxysubstituenten der Aryl-, Aralkyl- und Aralkenylreste sind Reste mit 1-4 C-Atomen bevorzugt, insbesondere die Methyl-, Ethyl-, iso-Butyl- und tert.-Butyl-Gruppe sowie die Methoxygruppe.

Als Aralkylreste $R^1$ kommen solche in Frage, deren geradkettiger oder verzweigter Alkylenanteil 1-5 Kohlenstoffatome enthält. Bevorzugte Aralkylreste $R^1$ sind der Phenethyl- und der 4-Chlor-phenethyl-Rest.

Unter Aralkenylresten $R^1$ sind solche zu verstehen, deren Alkenylenanteil 2-3 Kohlenstoffatome enthält. Hier sind bevorzugt der Styrylrest und der 4-Chlor-styryl-Rest.

Unter Halogen ist in allen Fällen Fluor, Chlor und Brom zu verstehen.

Als Alkylgruppen $R^2$ kommen geradkettige oder verzweigte mit 1-16 C-Atomen in Frage, bevorzugt sind die Gruppen Methyl und Octyl.

Die Acylreste $R^2$ leiten sich von aliphatischen Carbonsäuren mit 2-16 C-Atomen, von araliphatischen und von aromatischen Carbonsäuren ab. Bevorzugte Acylgruppen sind Acetyl, Isobutyroyl, Cinnamoyl, Benzoyl, 4-Chlor-benzoyl und 4-Aminobenzoyl sowie n-Octanoyl und n-Hexadecanoyl.

Die niederen Alkylgruppen $R^3$ und $R^4$ können geradkettig oder verzweigt sein und enthalten 1-6, vorzugsweise 1-4 Kohlenstoffatome, wobei die Methyl- und Ethylgruppe bevorzugt sind.

n bedeutet bevorzugt die Zahl 2.

$R^5$ ist bevorzugt ein Wasserstoffatom, oder bildet mit $R^6$ zusammen eine Alkylenkette mit 3 oder 4 C-Atomen, d.h., es liegt ein 5- oder 6-Ring vor, welcher ein Stickstoffatom enthält. Bevorzugt sind hier Verbindungen mit 5-Ring.

$R^6$ bedeutet bevorzugt ein Wasserstoffatom, oder ist eine Alkylkette mit 1 bis 4 C-Atomen, welche ggf. endständig substituiert ist. Als bevorzugte Substituenten sind zu nennen:

Carboxyl, Aminocarbonyl, Di-$C_1$-$C_6$-alkylaminocarbonyl, 4-subst. Piperazin-1-yl-carbonyl (der 4-ständige Substituent sei Benzyl oder 4-Chlor-benzyl), $C_1$-$C_6$-Alkoxycarbonyl, $C_1$-$C_6$-Alkylthio, $C_6$-$C_{14}$-Aryl-$C_1$-$C_4$-alkyl-thio, Phenylthio, Hydroxyl, Phenyl und 4-Imidazolyl, wobei die in diesen Substituenten ggf. enthaltenen Phenylkerne auch 1-2 Halogenatome (bevorzugt Chlor) enthalten können.

Die besonders bevorzugten Substituenten sind: Carboxyl, Aminocarbonyl, Diethylaminocarbonyl, 4-Benzyl- bzw. 4-(4-Chlor-benzyl)piperazin-l-yl-carbonyl, Methoxy- bzw. Ethoxy-carbonyl, Methylthio, Benzyl- bzw. (4-Chlorbenzyl)-thio, Phenyl- bzw. (4-Chlorphenyl)-thio, Hydroxyl, Phenyl bzw. 4-Chlor-phenyl und 4-Imidazolyl.

Insbesondere sind in der Gruppe

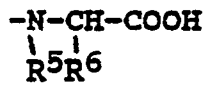

solche Reste $R^5$ und $R^6$ bevorzugt, deren Bedeutung die Struktur einer essentiellen Aminosäure ergibt. Dazu gehören auch alle möglichen Isomere und deren Gemische.

Zu den Aminosäuren zählen insbesondere Alanin, Arginin, Asparagin, Asparaginsäure, Cystein, Cystin, Glutamin, Glutaminsäure, Glycin, Histidin, Isoleucin, Leucin, Lysin, Methionin, Phenylalanin, Prolin, Serin, Threonin, Tryptophan, Tyrosin, Valin, Homocystein, Homoserin, Hydroxylysin, Hydroxyprolin, Ornithin, Sarkosin, Norvalin oder 2-Aminobuttersäure.

Als Ester der Carbonsäuren der allgemeinen Formel I kommen solche mit niederen einwertigen Alkoholen (wie z.B. Methanol oder Ethanol) oder mit mehrwertigen Alkoholen (wie z.B. Glycerin) in Frage, es seien aber auch solche Alkohole eingeschlossen, die noch andere funktionelle Gruppen enthalten, wie z.B. Ethanolamin.

Als Amide der Carbonsäuren der allg. Formel I werden solche beansprucht, in denen die Aminkomponente z.B. Ammoniak, ein niederes Dialkylamin wie z.B. Diethylamin oder ein Hydroxyalkylamin wie z.B. Ethanolamin oder Diethanolamin ist. Andere beanspruchte Aminkomponenten sind Alkyl-, Aralkyl- und Arylpiperazine.

Die Erfindung betrifft auch die reinen optischen Isomere (Enantiomere) der Verbindungen der allg. Formel I sowie deren Gemische (Razemate).

Besonders bevorzugte Verbindungen der Formel I sind Verbindungen, in denen $R_1$ Phenyl oder ein ein- oder zweifach durch Halogen, Methyl, Methoxy oder Trifluormethyl substituierten Phenyl, $R^2$, $R^3$, $R^4$ Wasserstoff, n die Zahl 2 und die Gruppe

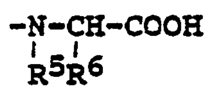

der Rest einer essentiellen Aminosäure oder deren optische Isomere bzw. Gemische daraus darstellt, wobei die Sulfonylaminoalkylgruppe bevorzugt in meta-Stellung zur Phenoxyalkylcarbonamid-Gruppe steht.

Die Herstellung der Phenoxycarbonsäureamide der allgemeinen Formel I ist dadurch gekennzeichnet, daß man

a) ein Amin der allg. Formel II

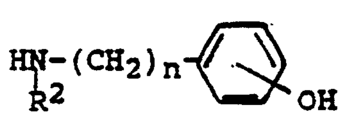

(II)

in welcher $R^2$ und n die oben angegebene Bedeutung haben, gegebenenfalls unter intermediärem Schutz der Amino- bzw. Hydroxylgruppe in an sich bekannter Weise in beliebiger Reihenfolge mit einer Sulfonsäure der allgemeinen Formel III

$$R^1-SO_2OH \quad (III)$$

in welcher $R^1$ die oben angegebene Bedeutung hat, bzw. einem Derivat derselben und mit einer gegebenenfalls optisch aktiven Verbindung der allgemeinen Formel IV

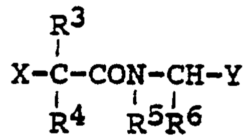

(IV)

bzw. mit einem Derivat desselben umsetzt. In Formel IV haben $R^3$, $R^4$ die oben angegebene Bedeutung, X stellt eine reaktive Gruppe dar, und unter Y ist eine Gruppe $COOR^7$ (wobei $R^7$ ein Wasserstoffatom oder ein Äquivalent eines Metallions, oder eine niedere Alkyl-, Aralkyl- bzw. Silylgruppe darstellt) oder eine Säureamidgruppe zu verstehen. Y kann aber auch einen Rest darstellen, der nach erfolgter Kondensation in eine Säureamidgruppe oder in die $-COOR^7$-Gruppe überführt wird, worauf man gegebenenfalls einen bestimmten Substituenten $R^7$ in an sich bekannter Weise in einen anderen Substituenten $R^7$ umwandelt und die erhaltenen Verbindungen in pharmakologisch unbedenkliche Salze überführt.
Das erfindungsgemäße Verfahren führt man zweckmäßig in zwei Stufen durch. Die Kondensation der Verbindungen der allgemeinen Formel II mit Sulfonsäuren III oder deren Derivaten einerseits und Verbindungen der allgemeinen Formel IV andererseits wird vorzugsweise so durchgeführt, daß man zunächst eine der beiden reaktiven Gruppen der Verbindung II mit einer leicht abspaltbaren Schutzgruppe blockiert, die erhaltene Verbindung mit einer Sulfonsäure III oder einem Derivat davon bzw. mit einer Verbindung der allgemeinen Formel IV umsetzt, die Schutzgruppe wieder abspaltet und anschließend dieses reaktive Zwischenprodukt mit der noch nicht eingesetzten Verbindung der allgemeinen Formel IV bzw. III umsetzt. Bevorzugt wird ein Verfahrensweg, bei dem die an der Aminogruppe geschützte Verbindung II (d.i. die Verbindung V) zunächst mit einer Verbindung IV zur Umsetzung gebracht wird. Nach Abspaltung der Schutzgruppe erfolgt dann die Reaktion mit einer Sulfonsäure III bzw. mit einem ihrer Derivate:

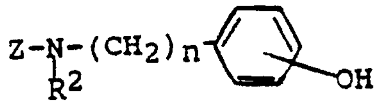

+

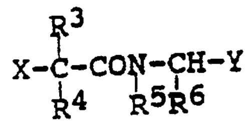

(V) (IV)

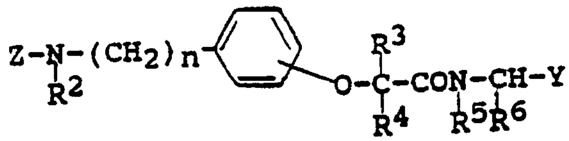

(VI)

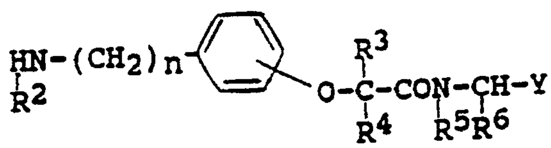

(VII)

$+R^1-SO_2OH$

(III)

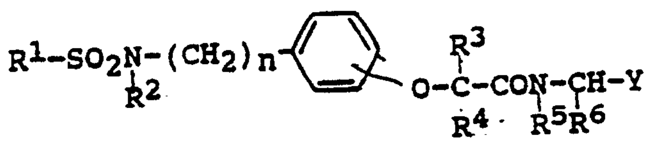

(VIII)

Die in den voranstehenden Formeln verwendeten Symbole $R^2$, n, X, $R^3$, $R^4$, $R^5$, $R^6$ und Y haben die oben angegebene Bedeutung. Z repräsentiert eine für den Schutz von Aminogruppen geeignete Gruppe. Die Herstellung der Zwischenverbindung VI kann auch in mehreren Teilschritten vollzogen werden: Man setzt zunächst V mit einer Carbonsäure IX, deren Carboxylfunktion zweckmäßig durch Derivatbildung geschützt ist, um:

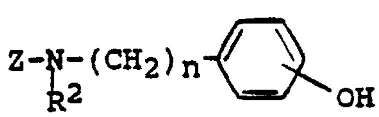

+

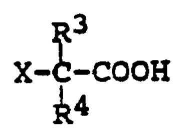

(V) (IX)

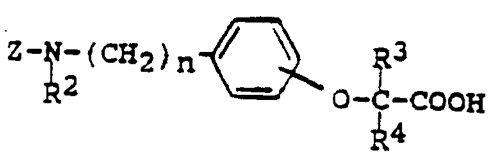

(X)

Die Phenoxyalkylcarbonsäure X wird nun (ggf. nach vorheriger Abspaltung der Carboxylschutzgruppe) mit einem gegebenenfalls optisch aktiven Amin der allg. Formel XI

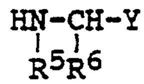

(XI)

oder einen Salz des Amins kondensiert. An Stelle der freien Carbonsäure werden zweckmäßigerweise reaktive Derivate zur Umsetzung gebracht. Die entstandene Verbindung VI wird dann, wie beschrieben, von der Aminschutzgruppe befreit und mit einer Sulfonsäure III umgesetzt.

Für den Fall, daß $R^3$ und $R^4$ niedere Alkylgruppen bedeuten, kann man die Phenoxyalkylcarbonsäuren X auch herstellen, indem man das Phenol V mit einem Gemisch aus einem aliphatischen Keton, Chloroform und einem Alkalihydroxid umsetzt. Vorzugsweise wird diese Variante zur Herstellung von Phenoxyisobuttersäure-Derivaten eingesetzt:

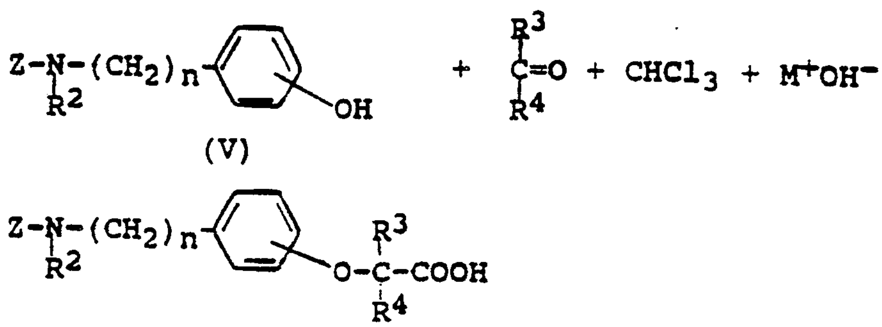

(V)

(X)

Dieses Verfahren ist auch anwendbar, wenn Z schon durch eine Gruppe $R^1$-$SO_2$- ersetzt ist.

b) Eine weitere Möglichkeit zur Herstellung von Verbindungen der allgemeinen Formel I, ihrer Salze sowie ihrer Ester und Amide besteht darin, daß man in Sulfonamid der allgemeinen Formel XI

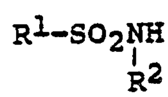

(XII)

mit einer gegebenenfalls optisch aktiven Verbindung der allgemeinen Formel XIII

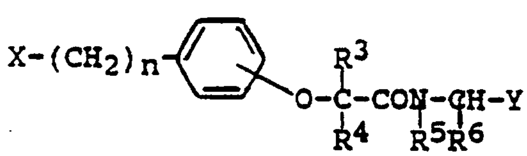

(XIII)

zur Umsetzung bringt und die Gruppe Y der entstandenen Verbindung VIII ggf. in die Carboxylfunktion oder eine andere gewünschte Funktion umwandelt.

Für den Fall, daß $R^2$ einen Acylrest darstellt, kommt bevorzugt die nachträgliche Acylierung einer solchen Verbindung I infrage, in welcher $R^2$ ein Wasserstoffatom darstellt und deren Carboxylfunktion verestert ist. Als Acylierungsmittel kommen reaktive Derivate von Carbonsäuren, bevorzugt Säurechloride, infrage.

Als reaktive Derivate der Sulfonsäuren III kommen insbesondere die Halogenide sowie die Ester in Betracht. Die Umsetzungen der Sulfonsäurehalogenide mit Verbindungen der allgemeinen Formeln II bzw. VII erfolgen zweckmäßig unter Zusatz eines säurebindenden Mittels, wie z.B. Alkaliacetat, Natriumhydrogencarbonat, Natriumcarbonat, Natriumphosphat, Calciumoxid, Calciumcarbonat oder Magnesiumcarbont. Diese Funktion können aber auch organische Basen wie z.B. Pyridin oder Triethylamin übernehmen, wobei als inertes Lösungsmittel z.B. Ether, Benzol, Methylenchlorid, Dioxan oder ein Überschuß des tertiären Amins dient. Bei Einsatz anorganischer Säurebinder verwendet man als Reaktionsmedium z.B. Wasser, wäßriges Ethanol oder wäßriges Dioxan.

Für eine Umsetzung der Verbindung II mit der Verbindung IV hat es sich als vorteilhaft herausgestellt, zunächst die Aminogruppe der Verbindung II in eine geschützte Aminogruppe umzuwandeln. Als besonders geeignet sind hier die aus der Peptidchemie bekannten, z.B. durch Hydrierung leicht zu entfernenden Gruppen wie die Benzyloxycarbonyl-Gruppe. Geeignet sind auch Schutzgruppen wie die Phthalimidogruppe, die nach erfolgter Kondensation zwischen IV und V mittels Hydroxylamin in bekannter Weise leicht wieder gespalten werden können. Gelegentlich kann man auf die Wiederabspaltung ganz verzichten, indem man von vornherein die Gruppe $R^1$-$SO_2$- einführt. Als reaktive Verbindungen IV bzw. IX bzw. XIII kommen insbesondere solche infrage, bei denen X das Anion einer starken Säure, z.B. einer Halogenwasserstoff- oder Sulfonsäure darstellt. Die Reaktionen dieser reaktiven Verbindungen können sehr begünstigt werden, wenn man die jeweiligen Reaktionspartner in Form ihrer Salze (also: V in Form des Natrium- oder Kaliumphenolats; XII in Form des Natrium- oder Kaliumsalzes des Sulfonamids) eingesetzt. Die Umsetzung der reaktiven Verbindungen der allg. Formeln IV resp. IX bzw. XIII mit den Natrium- oder Kaliumsalzen der Verbindungen V bzw. XII erfolgt in Lösungsmitteln wie z.B. Toluol, Methylethylketon, Dimethylformamid oder Dimethylsulfoxid, vorzugsweise in der Wärme.

Für die Umsetzung der Carbonsäuren X mit den Aminen XI hat es sich als zweckmäßig erwiesen, die Carbonsäuren in Form reaktiver Derivate einzusetzen. Als solche kommen infrage die Säurehalogenide, -anhydride, -imidazolide, die gemischten Anhydride aus der Carbonsäure und einem Chlorameisensäureester, aktive Ester wie z.B. Nitrophenylester oder Hydroxyphthalimid- bzw. Hydroxysuccinimid-ester. Günstig ist auch die Kondensation zwischen freier Carbonsäure X und Aminkomponente XI in Gegenwart von wasserabspaltenden Mitteln wie Dicyclohexylcarbodiimid. Die Säurefunktion der Aminkomponente kann in vielen Fällen in der Salzform vorliegen, oft ist es aber günstiger, von der veresterten Aminocarbonsäure XI auszugehen, wobei sich als ganz besonders günstig der Einsatz der Trialkylsilylester erwiesen hat. Bei der Herstellung der Trialkylsilylester wird ggf. gleichzeitig die Aminogruppe silyliert, so daß N-Trialkylsilylaminosäure-trialkylsilylester resultieren. Diese können in gleicher Weise mit den aktivierten Carbonsäuren X kondensiert werden wie am Stickstoff nicht-silylierte Aminosäureester. Die Aminogruppe der Aminosäuren bzw. ihrer Ester kann ggf. auch in der Salzform zur Reaktion bebracht werden. Als Lösungsmittel kommen infrage: Wasser-Alkohol-Gemische (z.B. für die Umsetzung von Hydroxysuccinimidestern der Carbonsäuren X mit den Natriumsalzen der Aminosäuren XI (Y = -COONa); Methylenchlorid, Tetrahydrofuran, Dimethylformamid und, in Fällen geringer Löslichkeit, Dimethylsulfoxid.

c) Verbindungen der Formel I können auch durch Umsetzung von Verbindungen der Formel XIV oder deren reaktive Derivate

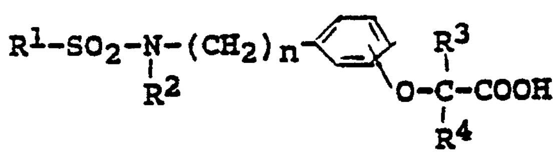

(XIV)

in der $R^1$, $R^2$, $R^3$, $R^4$ und n die angegebene Bedeutung haben, mit einer Aminosäure der Formel XI erhalten werden.

Die Verbindungen der Formel XIV sind teilweise in der DE-OS 36 10 643 sowie DE-OS 28 09 377 beschrieben bzw. können nach den dort beschriebenen Verfahren hergestellt werden.

Reaktive Derivate der Verbindungen der Formel XIV sind vorzugsweise Säurehalogenide, -Imidazolide oder gemischte Anhydride. Säurechloride werden in üblicher Weise aus den freien Säuren durch Umsetzung z.B. mit Thionylchlorid erhalten. Zur Herstellung von Verbindungen der allg. Formel VIII durch Alkylierung eines Sulfonamids XII mittels einer Verbindung XIII setzt man bevorzugt solche Verbindungen XIII ein, in denen X ein Halogenatom, d.h. Chlor oder Brom, darstellt. Bevorzugt ist ein

Umsetzungsverfahren, bei dem zwei Mol Sulfonamid XI mit einem Mol Natriumalkoholatlösung zur Trockne eingedampft werden. Dieses Gemisch wird nun mit einem Mol des Alkylhalogenids XIII umgesetzt. Auf diese Weise wird die Bildung dialkylierter Sulfonamide weitestgehend vermieden.

Die gegebenenfalls nachträgliche N-Alkylierung einer Verbindung der allgemeinen Formel VIII, in der $R^2$ = Wasserstoff bedeutet, kann nach bekannten Methoden durchgeführt werden, vorzugsweise, indem man sie mit einem Alkylhalogenid oder einem Dialkylsulfat in Gegenwart eines säurebindenden Mittels wie z.B. Kaliumcarbonat umsetzt.

Die Einführung einer Acylgruppe $R^2$ in ein Sulfonamid der allgemeinen Formel VIII ($R^2$ = H) erfolgt unter Bedingungen, wie sie für die Acylierung von Aminen üblich sind: Umsetzen mit einem aktiven Carbonsäure-Derivat, z.B. einem Säurehalogenid, einem gemischten Anhydrid oder einem aktiven Ester, in einem inerten Lösungsmittel in Gegenwart von Basen. Als inerte Lösungsmittel kommen beispielsweise Methylenchlorid, Benzol, Di-methylformamid u.ä. in Frage.

Als Substituenten Y der allgemeinen Formel VIII, die in die $-COOR^7$-Gruppe überführt werden können, kommen beispielsweise die Nitril-, Carbaldehyd-, Hydroxymethyl-, Aminomethyl- und Formylgruppen infrage. Die gegebenenfalls im Anschluß an die Kondensation durchzuführende Umwandlung des Substituenten $R^7$ erfolgt beispielsweise durch Verseifung der Carbonsäureester ($R^7$ = Alkyl) zu den entsprechenden Carbonsäuren ($R^7$ = Wasserstoff) mit Mineralsäuren oder Alkali-/ bzw. Erdalkalihydroxiden in einem polaren Lösungsmittel wie z.B. Wasser, wäßrigem Methanol, wäßrigem Ethanol oder wäßrigem Dioxan, bei Eisbad-Temperaturen oder bei Temperaturen bis 40 °C. Die jeweiligen Bedingungen richten sich nach der Verseifbarkeit der im Molekül vorhandenen Amidbindung.

Umgekehrt kann man aber auch die Carbonsäuren ($R^7$ = H) in üblicher Weise verestern oder Ester mit einem bestimmten Rest $R^7$ durch Umestern in einen Ester mit einem anderen Rest $R^7$ umwandeln. Die Veresterung der Carbonsäuren wird zweckmäßig in Gegenwart eines sauren Katalysators, wie z.B. Chlorwasserstoff, Schwefelsäure, p-Toluolsulfonsäure oder eines starken sauren Ionenaustauschharzes, vorgenommen. Umesterungen hingegen erfordern den Zusatz einer geringen Menge einer basischen Substanz, z.B. eines Alkali- oder Erdalkalihydroxids oder eines Alkalialkoholats. Für die Veresterung der Carboxygruppe bzw. für eine Umesterung eignen sich prinzipiell alle Alkohole. Bevorzugt sind die niederen einwertigen Alkohole wie Methanol, Ethanol oder Propanol, sowie mehrwertige Alkohole, z.B. Glycerin oder Alkohole mit anderen funktionellen Gruppen, wie Ethanolamin oder Glycerin-ether.

Die erfindungsgemäßen, von den Carbonsäuren der allgemeinen Formel I abgeleiteten Amide werden bevorzugt nach an sich bekannten Methoden aus den Carbonsäuren oder ihren reaktiven Derivaten (wie z.B. Cabonsäurehalogeniden, -estern, -aziden, -anhydriden oder gemischten Anhydriden) durch Umsetzung mit Aminen hergestellt. Als Aminokomponenten kommen z.B. Ammoniak, Alkylamine, Dialkylamine, aber auch Aminoalkohole wie z.B. Ethanolamin und 2-Aminopropanol infrage. Andere wertvolle Aminkomponenten sind Alkyl-, Aralkyl- und Arylpiperazine, z.B. Benzylpiperazin. Zur Herstellung von Salzen mit pharmakologisch verträglichen organischen oder anorganischen Basen, wie z.B. Natriumhydroxid, Kaliumhydroxid, Calciumhydroxid, Ammoniumhydroxid, Methylglukamin, Morpholin oder Ethanolamin können die Carbonsäuren mit den entsprechenden Basen umgesetzt werden. Auch Mischungen der Carbonsäuren mit einem geeigneten Alkalicarbonat bzw. -hydrogencarbonat kommen in Betracht.

Zu reinen Enantiomeren der Verbindungen der Formel I kommt man entweder durch Razematspaltung (über Salzbildung mit optisch aktiven Basen), oder indem man in die Synthesen gemäß Verfahren a) bis c) jeweils optisch reine Aminosäuren einsetzt.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat-oder Borat-Puffer, Ethanol, Dimethylsulfoxid, Komplexbildner (wie Ethylendiamin-tetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Fettsäuren (wie Stearinsäure), Gelantine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem

Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0,1 bis 50 mg/kg Körpergewicht. Normalerweise sind 0,5 bis 40 und vorzugsweise 1,0 bis 20 mg/kg/Tag in einer oder mehreren Anwendungen pro Tag wirksam, um die gewünschten Resultate zu erhalten.

Bevorzugt im Sinne der Erfindung sind außer den in den Beispielen genannten Verbindungen der Formel I sowie deren Estern, Amiden und Salzen die folgenden:

1. N-[3-[2-(Phenylsulfonylamino)ethyl]phenoxyacetyl]glycin
2. N-[2-[2-(4-Chlor-phenylsulfonylamino)ethyl]phenoxyacetyl]alanin
3. N-[3-[2-(4-Methyl-phenylsulfonylamino)ethyl]phenoxyacetyl] alanin
4. N-[3-[2-(3-bzw.4-Trifluormethyl-phenylsulfonylamino]ethyl] phenoxyacetyl]-2-amino-buttersäure
5. N-[3-[2-(4-Chlor-phenylsulfonylamino)ethyl]phenylacetyl]alanin-(4-benzylpiperazid)
6. N-[3-[2-(4-Methoxy-phenylsulfonylamino)ethyl]phenoxyacetyl] alanin-ethylester
7. N-[3-[2-(2.4-bzw.2.5-Dichlor-phenylsulfonylamino)ethyl]phenoxyacetyl]alanin
8. N-[4-[2-(3.5-Dichlor-phenylsulfonylamino)ethyl]phenoxyacetyl-L-alanin
9. N-[4-[2-(4-Trifluormethyl-phenylsulfonylamino)ethyl]phenoxyacetyl-L-alanin
10. N-[4-[2-(4-Chlor-phenylsulfonylamino)ethyl]phenoxyacetyl-L-serin
11. N-[4-[2-(4-Trifluormethyl-phenylsulfonylamino)ethyl]phenoxyacetyl-L-serin
12. N-[4-[2-(4-Methyl-phenylsulfonylamino)ethyl]phenoxyacetylglycin

Die nachfolgenden Beispiele zeigen einige der zahlreichen Verfahrensvarianten, die zur Synthese der erfindungsgemäßen Verbindungen verwendet werden können. Sie sollen jedoch keine Einschränkung des Erfindungsgedankens darstellen.

Beispiel 1

N-[3-[2-(4-Chlor-phenylsulfonylamino)ethyl]phenoxyacetyl]-glycin

a) 3-[3-(4-Chlor-phenylsulfonylamino)ethyl]phenoxy acetylchlorid

Man rührt ein Gemisch aus 30 g (81 mmol) 3-[2-(4-Chlorphenylsulfonylamino)ethyl]phenoxyessigsäure, 23,5 ml (0,32 mol) Thionylchlorid und 3 Tropfen DMF zwei Stunden bei 60° C und destilliert anschließend überschüssiges Thionylchlorid im Vacuum ab. Der Rückstand wird in abs. Ether gelöst und mit Aktivkohle geklärt. Nach dem Eindampfen erhält man ein langsam durchkristallisierendes Öl. Man verrührt mit Isohexan, saugt ab und trocknet. Ausb. 26,9 g (94 % d.Th.), Schmp. 75-77° C.

b) Titelverbindung

Zu einer eiskalten Lösung aus 0,8 g (10 mmol) Glycin und 15 ml 2N NaOH tropft man langsam eine Lösung aus 3,52 g des nach a) erhaltenen Säurechlorids und 40 ml Methylenchlorid und läßt anschließend zwei Stdn. bei 0° C nachreagieren. Man säuert nun mit verd. HCl an und schüttelt die schwach saure Phase mit Essigester aus. Die organ. Phase wird zweimal mit 1N-HCl extrahiert, mit Wasser gewaschen und mit $Na_2SO_4$ getrocknet. Das nach dem Eindampfen erhaltene Rohprodukt wird aus Nitromethan umkristallisiert. Ausb. 2,7 g (63 % d.Th.), Schmp. 134-135° C.

Beispiel 2

N-[3-[2-(4-Brom-phenylsulfonylamino)ethyl]phenoxyacetyl]-L-alanin

a) N-Trimethylsilyl-L-alanin-trimethylsilyl-ester

Zu einer Suspension aus 7,12 g (80 mmol) L-Alanin und 120 ml abs. $CH_2Cl_2$ tropft man unter Rühren 20,8 ml Trimethylchlorsilan und erhitzt anschließend kurz auf Rückflußtemperatur, wobei eine klare Lösung entsteht. Dann werden unter leichtem Kühlen 22,4 ml Triethylamin unter Rühren so zugetropft, daß der Ansatz nicht zu heftig siedet. Anschließend erhitzt man den Kolbeninhalt, eine dicke Suspension, 5 min lang auf Rückflußtemperatur, kühlt ab, filtriert durch ein Druckfilter und wäscht anschließend mit trocknem $CH_2Cl_2$ nach. Das Produkt wird im Stickstoffstrom getrocknet und roh weiterverarbeitet.

b) Titelverbindung

Zu einer Lösung aus 9,28 g (20 mmol) 3-[2-(4-Bromphenylsulfonylamino)ethyl]phenoxyacetyl-imidazol in 75 ml abs. THF gibt man bei -5 °C unter Feuchtigkeitsausschluß 5,14 g (22 mmol) der nach a) erhaltenen Trimethylsilylverbindung, läßt im Laufe von drei Stdn. auf Raumtemperatur kommen und destilliert anschließend i. Vak. das THF ab. Der Rückstand wird mit 2N HCL bei Raumtemperatur gerührt, dann extrahiert man mehrfach mit Methylenchlorid. Die vereinten organischen Phasen werden mit einer zur Salzbildung ausreichenden Menge 2N-$NaHCO_3$-Lösung extrahiert, und der Extrakt wird angesäuert. Das ausfallende Produkt wird abgesaugt, getrocknet und aus Nitromethan umkristallisiert. Ausb. 6,2 g (60 % d.Th.), Schmp. 191-192 °C.

Beispiel 3

N-[3-[2-(4-Chlor-phenylsulfonylamino)ethyl]phenoxyacetyl]-L-alanin

a) N-[3-[2-(Benzyloxycarbonylamino)ethyl]phenoxyacetyl]-L-alanin

Zu einer 40 °C warmen Lösung aus 5,0 g (15,8 mmol) 3-[2-(Benzyloxycarbonylamino)ethyl]-phenoxyessigsäure und 25 ml abs. DMSO gibt man 2,56 g (15,8 mmol) Carbonylbisimidazol und rührt weitere 30 min bei 40 °C. Anschließend setzt man 1,74 g (15,8 mmol) L-Alanin-natriumsalz zu, hält drei Stdn. auf 60 °C, kühlt ab und rührt in ein Gemisch aus Eis und so viel Salzsäure ein, daß die Mischung etwa pH2 erreicht. Das ausfallende Öl wird mit $CH_2Cl_2$ extrahiert. Nach Waschen der $CH_2Cl_2$-Phase mit $NaHCO_3$-Lösung und Wasser wird sie getrocknet ($MgSO_4$) und eingedampft. Man erhält 5,21 g (82 % d.Th.) Produkt in Form eines farblosen Oels.

Alternativsynthese für diese Verbindung:

Man hält ein Gemisch aus 22,0 g (81 mmol) 3-[2-(Benzyloxycarbonylamino)ethyl]phenol, 200 ml Butanon und 33,5 g feinst pulverisiertem trockenem $K_2CO_3$ eine Stunde lang auf Rückflußtemperatur, gibt 300 mg Kaliumiodid und 17,3 g (89,3 mmol) N-(2-Chloracetyl)-L-alanin-ethylester zu und hält nun 16 Stdn. auf Rückflußtemperatur. Anschließend wird abgesaugt und der Filterkuchen mit heißem Butanon nachgewaschen. Man dampft die vereinten Butanonphasen ein, nimmt den öligen Rückstand in $CH_2Cl_2$ auf, wäscht die Methylenchloridphase mit Wasser, trocknet ($MgSO_4$) und dampft i.Vak. ein. Ausbeute: praktisch quantitativ.

b) Titelverbindung

Man hydriert ein Gemisch aus 8,0 g (20 mmol) der nach a) erhaltenen Benzyloxycarbonyl-Verbindung, 75 ml Methanol, 11,0 ml 2N-HCl und ca. 2 g 10-proz. Palladiumkohle 12 h lang bei Raumtemperatur und 6 bar, bis die erforderliche Menge Wasserstoff aufgenommen ist. Dann wird der Katalysator abgesaugt und im Vacuum das Methanol abdestilliert. Zu dem Rückstand, welcher aus rohem Hydrochlorid des N-[3-(2-Aminoethyl)phenoxyacetyl]-L-alanins besteht, gibt man 75 ml Wasser und so viel 2N-NaOH, daß ph10 erreicht wird. Unter Rühren bei 0 °C gibt man nun 3,16 g 4-Chlor-benzolsulfochlorid in kleinen Anteilen zu und sorgt durch Zutropfen weiterer verd. NaOH dafür, daß pH10 erhalten bleibt. Anschließend wird zwei Stdn. bei 35 °C nachgerührt. Man säuert an, saugt ab, trocknet und kristallisiert aus Nitromethan um. Aus. 4,7 g (71 % d.Th.), Schmp. 175-177 °C.

Beispiel 4

N-[3-[2-(4-Chlor.-phenylsulfonylamino)ethyl]phenoxyacetyl]-L-alanin

a) N-[3-[2-(4-Chlor-phenylsulfonylamino)ethyl]phenoxyacetyl]-L-alanin-ethylester

Zu einem eiskalten Gemisch aus 2,2 g (14 mmol) L-Alanin-ethylester-hydrochlorid, 4,3 g Triethylamin und 100 ml Methylenchlorid tropft man langsam unter Rühren eine Lösung aus 5,0 g (14 mmol) 3-[2-(4-Chlor-phenylsulfonylamino)ethyl]-phenoxyacetylchlorid und 25 ml Methylenchlorid. Anschließend wird eine Std. bei 0 °C und dann eine Std. bei 25 °C gerührt, mit verd. HCl und Wasser ausgeschüttelt, getrocknet ($Na_2SO_4$) und schließlich eingedampft. Aus. 5,1 g (77 % d.Th.). Farbloses Oel.

b) Titelverbindung

Ein Gemisch aus 2,8 g (6 mmol) des nach a) erhaltenen Esters, 25 ml Ethanol und 15 ml 2N-NaOH wird

fünf Minuten bei Eisbadtemperatur gerührt, danach mit 25 ml Eiswasser verdünnt und mit verd. HCl angesäuert. Nach Absaugen, Trocknen und Umkristallisieren aus Nitromethan Ausb. 1,9 g (72 % d.Th.). Schmp. 175-177 °C. Das Produkt ist mit dem nach Beispiel 3 erhaltenen identisch.

Beispiel 5

N-[3-[2-(4-Chlor-phenylsulfonylamino)ethyl]phenoxyacetyl]-L-methionin

Zu einer Lösung aus 8,0 g (20 mmol) 3-[2-(4-Chlor-phenyl-sulfonylamino)ethyl]phenoxyessigsäure und 35 ml DMSO gibt man bei 40 °C unter Rühren 3,5 g (20 mmol) Carbonylbisimidazol, wobei Schaumbildung auftritt. Nach einer Stunde Rühren bei 40 °C gibt man 4,07 g (24 mmol) L-Methionin-Natriumsalz zu und hält nun 18 Stdn. bei 60 °C. Dann wird abgekühlt, auf ein Eis-Salzsäuregemisch gegossen und das schmierig ausfallende Produkt in Essigester aufgenommen. Die Essigesterphase wird mit Sodalösung extrahiert und letztere wiederum mit Essigester gewaschen. Man säuert die wäßrige Phase an, saugt ab, wäscht mit Wasser, trocknet und kristallisiert aus Nitromethan um. Ausb. 8,3 g (77 % d.Th.). Schmp. 138-139 °C.

In analoger Weise erhält man aus 3-[2-(4-Chlor-phenylsulfonylamino)ethyl]phenoxyessigsäure die folgenden Verbindungen:

2. - mit L-Histidin-Natriumsalz:

N-[3-[2-(4-Chlor-phenylsulfonylamino)ethyl]phenoxyacetyl]-L-histidin-hydrochlorid.
Ausb. 34 %. Schmp. 100-103 °C.

Das nach dem Eingießen in Eis-Salzsäuregemisch ausfallende ölige Produkt wird hier mit einem Aceton-Ether-Gemisch (1:12 Vol.) verrieben, bis es kristallisiert. Dann saugt man schnell ab (hygroskopisch!) und trocknet über $P_2O_5$.

3. - mit L-2-Aminobuttersaurem Natrium:

N-[3-[2-(4-Chlor-phenylsulfonylamino)ethyl]phenoxyacetyl]-L-2-aminobuttersäure. Aus. 61 % d.Th., Schmp. 147-150 °C (Nitromethan).

4. - mit L-Asparagin-Natriumsalz:

N-[3-[2-(4-Chlor-phenylsulfonylamino)ethyl]phenoxyacetyl]-L-asparagin.
Ausb. 62 % d.Th., Schmp. 133-135 °C (Nitromethan).

5. - mit L-Phenylalanin-Natriumsalz:

N-[3-[2-(4-Chlor-phenylsulfonylamino)ethyl]phenoxyacetyl]-L-phenylalanin.
Ausb. 63 % d.Th., Schmp. 158-159 °C (wäßr. Ethanol).

6. - mit L-Prolin-Natriumsalz:

N-[3-[2-(4-Chlor-phenylsulfonylamino)ethyl]phenoxyacetyl]-L-prolin.
Ausb. 93 % d.Th., Schmp. 114-116 °C (Essigester)

7. - mit L-Serin-Natriumsalz:

N-[3-[2-(4-Chlor-phenylsulfonylamino)ethyl]phenoxyacetyl]-L-serin.
Ausb. 49 % d.Th., Schmp. 146-148 °C (Nitromethan).

8. - mit L-Norvalin-Natriumsalz:

N-[3-[2-(4-Chlor-phenylsulfonylamino)ethyl]phenoxyacetyl]-L-norvalin.
Ausb. 72 % d.Th., Schmp. 126-128 °C (Nitromethan).

Beispiel 6

N-[3-[2-(4-Chlor-phenylsulfonylamino)ethyl]phenoxyacetyl]-D-alanin

In einer 40° C warmen Lösung aus 10,0 g (27 mmol) 3-[2-(4-Chlorphenylsulfonylamino)ethyl]-phenoxyessigsäure und 30 ml DMSO gibt man 4,38 g (27 mmol) Carbonylbisimidazol, läßt 10 min reagieren, fügt 3,76 g (27 mmol) 4-Nitrophenol zu und rührt weitere 10 min. Dann trägt man 3,0 g (27 mmol) D-Alanin-Natriumsalz ein und rührt drei Stdn. bei 60° C. Nach dem Abkühlen wird der Ansatz in ein Eis-Salzsäure-Gemisch eingerührt. Man nimmt das ausgefallene Öl in Essigester auf, wäscht die orga. Phase mit Wasser, trocknet ($Na_2SO_4$) und dampft ein. Der ölige Rückstand kristallisiert nach Zugabe von Isohexan. Ausb. 8,0 g (67 % d.Th.), Schmp. 163-166° C.

Beispiel 7

In einer zu Beispiel 6 analogen Verfahrensweise wurden dargestellt aus

4-[2-(Phenylsulfonylamino)ethyl]phenoxyessigsäure

a) und L-Alanin:

N-[4-[2-(Phenylsulfonylamino)ethyl]phenoxyacetyl]-L-alanin
Ausb. 62 % d.Th., Schmp. 164-165° C.

b) und D-Alanin:

N-[4-[2-(Phenylsulfonylamino)ethyl]phenoxyacetyl]-D-alanin Ausb. 54 % d.Th., Schmp. 152-153° C.

## Ansprüche

**1.** Verbindungen der Formel I

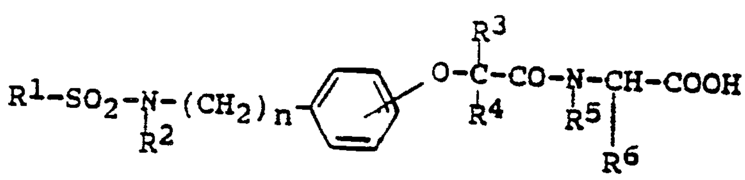

(I)

in welcher die Sulfonylaminoalkylgruppe in ortho-, meta- oder para-Stellung zur Phenoxyalkylcarbonamid-Gruppe steht und in welcher

| | |
|---|---|
| $R^1$ | eine Aryl-, Aralkyl oder eine Aralkenylgruppe bedeutet, deren Arylrest jeweils ein- oder mehrfach durch Halogen, Cyan, Alkyl, Trifluormethyl oder Alkoxy substituiert sein kann, |
| $R^2$ | Wasserstoff, eine Alkyl- oder eine Acylgruppe darstellt, |
| $R^3$ und $R^4$, | die gleich oder verschieden sein können, Wasserstoff oder eine niedere Alkylgruppe und |
| n | die Zahlen 1-3 bedeuten und worin |
| $R^6$ | Wasserstoff oder eine niedere Alkylgruppe mit 1-4 C-Atomen bedeutet, welche ggf. endständig durch Carboxyl, Aminocarbonyl, Alkoxycarbonyl, durch Alkylthio-, Hydroxyl, Phenyl oder durch eine Imidazol-4-ylgruppe substituiert ist und |
| $R^5$ | Wasserstoff bedeutet, oder zusammen mit $R^6$ eine Alkylenkette mit 3 oder 4 C-Atomen bildet, sowie deren physiologisch unbedenkliche Salze, Ester und Amide, und optische Isomere. |

**2.** Verbindungen der Formel I gemäß Anspruch 1, in denen $R^1$ Phenyl oder ein- oder zweifach durch Halogen, Methyl, Methoxy oder Trifluormethyl substituiertes Phenyl, $R^2$, $R^3$, $R^4$ Wasserstoff, n die Zahl 2 und die Gruppe

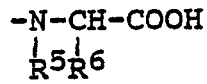

den Rest einer essentiellen Aminosäure oder deren optische Isomere bzw. Gemische daraus darstellt, wobei die Sulfonylaminoalkylgruppe in meta-Stellung zur Phenoxyalkylcarbonamid-Gruppe steht.

3. Verbindungen gemäß Anspruch 1 oder 2, in denen die Gruppe

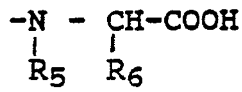

den Rest einer Aminosäure darstellt, ausgewählt aus der Gruppe Glycin, Alanin, 2-Aminobuttersäure, Serin, Methionin, Asparigin, Phenylalanin, Prolin oder Norvalin.

4. Verfahren zur Herstellung von Verbindungen der Formel I

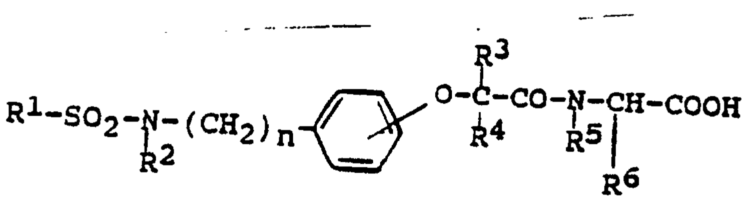

(I)

in welcher die Sulfonylaminoalkylgruppe in <u>ortho</u>-, <u>meta</u>- oder <u>para</u>-Stellung zur Phenoxyalkylcarbonamid-Gruppe steht und in welcher

| | |
|---|---|
| $R^1$ | eine Aryl-, Aralkyl oder eine Aralkenylgruppe bedeutet, deren Arylrest jeweils ein- oder mehrfach durch Halogen, Cyan, Alkyl, Trifluormethyl oder Alkoxy substituiert sein kann, |
| $R^2$ | Wasserstoff, eine Alkyl- oder eine Acylgruppe darstellt, |
| $R^3$ und $R^4$, | die gleich oder verschieden sein können, Wasserstoff oder eine niedere Alkylgruppe und |
| n | die Zahlen 1-3 |
| | bedeuten und worin |
| $R^6$ | Wasserstoff oder eine niedere Alkylgruppe mit 1-4 C-Atomen bedeutet, welche ggf. endständig durch Carboxyl, Aminocarbonyl, Alkoxycarbonyl, durch Alkylthio-, Hydroxyl, Phenyl oder durch eine Imidazol-4-ylgruppe substituiert ist und |
| $R^5$ | Wasserstoff bedeutet, oder zusammen mit $R^6$ eine Alkylenkette mit 3 oder 4 C-Atomen bildet, |

sowie deren physiologisch unbedenkliche Salze, Ester und Amide und optische Isomere,
dadurch gekennzeichnet, daß man

a) ein Amin der allg. Formel II

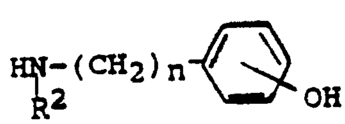

(II)

in welcher $R^2$ und n die oben angegebene Bedeutung haben, gegebenenfalls unter intermediärem Schutz der Amino- bzw. Hydroxylgruppe in an sich bekannter Weise in beliebiger Reihenfolge mit einer Sulfonsäure der allgemeinen Formel III

$$R^1\text{-}SO_2OH \qquad \text{(III)}$$

in welcher R[1] die oben angegebene Bedeutung hat, bzw. einem Derivat derselben und mit einer gegebenenfalls optisch aktiven Verbindung der allgemeinen Formel IV

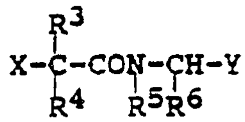

(IV).

in der $R_3$, $R_4$, $R_5$ und $R_6$ die angegebene Bedeutung haben, und X eine reaktive Gruppe und Y eine Gruppe COOH darstellt,

bzw. mit einem Derivat derselben umsetzt,
oder
b) ein Sulfonamid der Formel XI

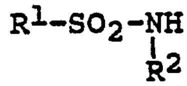

in der $R^1$ und $R^2$ die angegebene Bedeutung haben, mit einer Verbindung der Formel XIII

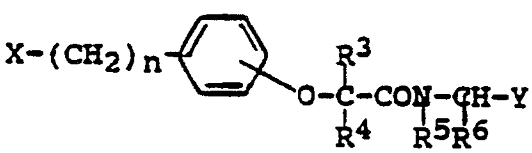

(XIII)

in der $R^3$, $R^4$, $R^5$, $R^6$, X, n und Y die angebene Bedeutung haben,
umsetzt oder
c) eine Verbindung der Formel XIV

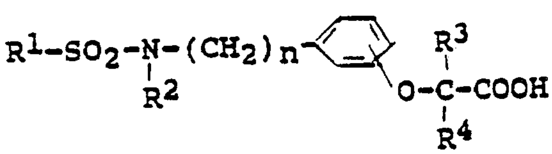

(XIV)

in der $R^1$, $R^2$, $R^3$, $R^4$ und n die angegebene Bedeutung haben, mit einem gegebenenfalls optisch aktiven Amin der Formel XI

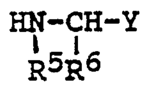

(XI)

in der $R^5$, $R^6$ und Y die angegebene Bedeutung haben umsetzt und anschließend die erhaltene Säure in physiologisch unbedenkliche Salze, Ester oder Amide bzw. erhaltene Ester oder Amide in freie Säuren überführt sowie erhaltene Racemate in die Enantiomeren spaltet.

5. Arzneimittl enthaltend eine Verbindung gemäß Anspruch 1, 2 oder 3 neben üblichen Träger- und Hilfsstoffen.

6. Verwendung von Verbindungen gemäß Anspruch 1, 2 oder 3 zur Behand-lung von Herz- und Kreislauferkrankungen.

Europäisches Patentamt

# EUROPÄISCHER RECHERCHENBERICHT

Nummer der Anmeldung

**EP 90 12 4076**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 325 245 (TANABE SEIYAKU CO., LTD)<br>* das ganze Dokument *<br>– – – | 1-6 | C 07 C 311/17<br>C 07 C 311/13<br>C 07 C 303/38<br>A 61 K 31/18<br>C 07 D 207/16<br>C 07 D 211/60 |
| A | EP-A-0 255 728 (TANABE SEIYAKU CO., LTD)<br>* das ganze Dokument *<br>– – – | 1,5,6 | |
| A | EP-A-0 223 593 (GLAXO GROUP LIMITED)<br>* das ganze Dokument *<br>– – – | 1,5,6 | |
| A,D | EP-A-0 239 907 (BOEHRINGER MANNHEIM GMBH)<br>* das ganze Dokument & DE-A-3610643 *<br>– – – | 1,5,6 | |
| A,D | EP-A-0 004 011 (BOEHRINGER MANNHEIM GMBH)<br>* das ganze Dokument & DE-A-2809377 *<br>– – – – – | 1,5,6 | |

RECHERCHIERTE SACHGEBIETE (Int. Cl.5)

C 07 C

Der vorliegende Recherchenbericht wurde für alle Patentansprüche erstellt

| Recherchenort | Abschlußdatum der Recherche | Prüfer |
|---|---|---|
| Berlin | 13 Februar 91 | RUFET J.M.A. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument